# EUROPEAN PATENT SPECIFICATION

(11) **EP 2 170 280 B1**
(45) Date of publication and mention of the grant of the patent: **20.03.2013**
(21) Application number: 08762490.4
(22) Date of filing: 25.06.2008
(51) Int. Cl.: A61K 9/12, A61K 31/465, A61K 33/00, A61P 11/00

(54) **AN INHALABLE COMPOSITION COMPRISING NICOTINE**
NIKOTIN ENTHALTENDE INHALIERBARE ZUSAMMENSETZUNG
COMPOSITION INHALABLE COMPRENANT DE LA NICOTINE

(30) Priority: 25.06.2007 GB 0712308
(43) Date of publication of application: 07.04.2010
(73) Proprietor: Kind Consumer Limited, London EC1R 5AR (GB)
(72) Inventor: HEARN, Alex, London SE1 3UW (GB); BAKRI, Sam, London SW1X 8LP (GB)
(74) Representative: Draper, Martyn John
(86) International application number: PCT/GB2008/002184
(87) International publication number: WO 2009/001085

(56) References cited:
- GB-A- 1 528 391
- US-A1- 2006 018 840

## Description

The present invention relates to a pressurised container comprising an inhalable composition comprising oxygen and nicotine or a salt or derivative thereof, and to said inhalable composition. The present invention also relates to an apparatus for nicotine delivery comprising a housing containing the composition and means for delivering the composition to the nasal or oral cavity of a user.

Tobacco smoke contains nicotine, a well-known stimulant. Nicotine is highly addictive and is the main reason why smokers find it difficult to give up smoking. The most harmful health effects of smoking, however, are attributable to the substances that are formed during the combustion of tobacco, such as carbon monoxide and tar.

A number of products have been developed to help smokers give up smoking. For example, nicotine replacement therapies have been developed to provide physiologically acceptable alternatives to tobacco smoke. Examples of such therapies include nicotine skin patches for transdermal administration of nicotine and nicotine-containing chewing gums and lozenges. These therapies, however, are limited by slow nicotine absorption profiles. Subsequently there is a growing need for faster acting nicotine delivery systems with better absorption profiles as highlighted in the UK Government paper on tobacco limitation 31st May 2008. Especially there is a need for devices that simulate the rapid take up of nicotine in a pharmacokinetically similar manner to the tobacco cigarette, and provide enhanced chemical formulations which reduce harm and have greater influence on the desire for the user to transfer from tobacco smoking. With the growing restrictions on the smoking of cigarettes in public places, there is room for a composition or device which can replace the physical act of smoking, which is socially acceptable such that it can be used in all public places. Also, there is a need for a device which can be used to dispense nicotine in a non-smokable form either as a cigarette replacement, or to address the nicotine dependency that smokers feel thereby helping them to give up smoking.

US 5,167,242 describes an inhaler comprising a cylindrical cartridge containing a reservoir of nicotine. Air inhaled through the cartridge becomes saturated with nicotine from the reservoir. Thus, when a user inhales air through the cartridge, he inhales a mixture of air and nicotine into his lungs. A disadvantage of the device is that there is a limit to the amount of nicotine that is administered through each inhalation. WO 2004/076289 attempts to address this problem by providing a container that allows a larger amount of air to be inhaled per puff. The container comprises a shaped mass of fibres or filaments that provide a reduced resistance to the flow of air inhaled through the container. The container may also contain additives, such as antioxidants, flavours and aromas. However, it has been found that the user still does not inhale enough nicotine per puff to provide the stimulant effect achieved with smoking. Moreover because the device is not pressurized, the device delivers most of the nicotine to the buccal and mucosal cavities and does not generate an aerosol plume of sufficient velocity in the inspiration flow for the majority of the aerosol to reach the lungs. This results in a much longer period for nicotine to reach acceptable levels in blood plasma concentrations, typically 3-7 minutes rather than between 7 -14 seconds. GB 1528391 relates to nicotine-containing aerosol compositions. US 2006/001884 A1 relates to an aerosolizable formulation comprising nicotine.

WO 01/49349 discloses an oxygen delivery apparatus in which oxygen is stored in a canister that is then removed from the device and is either refilled or replaced. The apparatus delivers quick or short breaths of pure oxygen to the user to supplement the user's oxygen supply, for example, during exercise. There is no indication in this reference that the apparatus can be used as a cigarette replacement, or to address the nicotine dependency that smokers feel.

The invention provides an apparatus according to claim 1 of the claims appended hereto.

According to a first reference example there is provided a pressurised container containing a composition comprising oxygen, nicotine or a nicotine derivative or salt thereof, and a solvent, wherein the container is pressurised to at least 3 x 10⁵Pa (3 bar), said container having an outlet valve which is selectively operable to release the composition from the container.

According to a second reference example is provided an inhalable composition comprising
at least 22 volume % oxygen, and
nicotine or a derivative or salt thereof.

The composition contained in the apparatus of the present invention may be delivered to the nasal or oral cavity of a user using any suitable apparatus. The composition is preferably dispensed as a mixture of aerosolised particles. Preferably, the composition has a particle size distribution that is similar to tobacco smoke. The composition may have the appearance of a vapour or smoke.

The concentration of oxygen in the composition of the apparatus the present invention is greater than the concentration of oxygen in the atmosphere. Accordingly, the compositiont contained in the apparatus of the present invention delivers an increased level of oxygen in combination with nicotine or a salt or derivative thereof. Advantages of the composition are explained in further detail below.

The increased levels of oxygen in the composition contained in the apparatus of the present invention may also increase the effectiveness of nicotine in its physiological functions. For example, it has been found that the psychoactive effect of nicotine is enhanced when it is delivered with increased levels of oxygen. This provides the user with increased stimulation and, in particular, increased cognitive stimulation. Oxygen is known to influence dopamine, a neurotransmitter, which is believed to act along the same reward pathways as nicotine. Oxygen has been found to be especially useful as an adjuvant in nicotine replacement therapy.

With the use of increased levels of oxygen, nicotine undergoes a partial degradation and oxidation. This is normally associated with the combustion in a tobacco cigarette. However, when nicotine is delivered with the increased levels of oxygen of the present invention, it undergoes a similar reactive change. Not only is this more acceptable to the user in terms of taste but, because it lessens the harsh pH of some of the acidic nicotine salts used in delivery, it also causes less irritation in the pulmonary and bronchial tracts, particularly the mucosal and buccal tract.

The composition contained in the apparatus of the present invention may be used in nicotine replacement therapy (NRT). For example, it may be delivered to NRT patients who are advanced smokers and are displaying early or advanced stages of emphysema or chronic obstructive pulmonary disease (COPD). By using oxygen instead of air, patients can enhance their oxygen absorption rates. This may be beneficial to their breathing patterns, allowing less reliance on generating deep inhalations. This is advantageous over air-delivered nicotine because the patient does not have to inhale so deeply to achieve his/her fill of nicotine, thereby reducing wheezing and/or COPD/asthma related attacks.

The increased levels of oxygen in the composition contained in the apparatus of the present invention can also quicken the rate of absorption and transportation of nicotine through the blood. It is believed that the increased levels of oxygen causes a higher degree of oxygenation in the blood of a user by the enhancing of plasma nicotine concentrations and therefore faster absorption across the blood brain barrier. This increases the speed and efficacy of nicotine reaching the receptors (e.g. nicotinic acetylcholine receptors) in the brain, thereby enhancing the targeting potential of nicotine as a replacement drug. In preferred embodiments, the composition contained in the apparatus of the present invention can deliver less nicotine per unit volume of inhalation, but can still achieve an equal, similar or improved satisfaction of nicotine craving for the user. This may be useful because a lower dose of nicotine, helps the user avoid some of the more dangerous vasoconstrictive effects of nicotine.

The composition contained in the apparatus of the present invention may also be used to treat diseases, such as neurodegenerative diseases like Alzheimer's and Parkinson's. The increased level of oxygen is believed to enrich the flow of oxygenated blood to the extremities of the brain. The increased blood flow and widening of the capillaries in the extremities of the brain allow the nicotine more contact with receptors, thereby increasing the efficacy of the beneficial psychoactive action on neurotransmitters around the brain. This effect may be enhanced when additives, such as gingko and gingko extracts, are included in the composition. In a preferred embodiment, therefore, the composition additionally includes gingko and/or a gingko derivative.

The composition contained in the apparatus of the present invention provides better anxiolytic effects than those achieved with conventional methods of nicotine delivery. As a result, the composition contained in the apparatus of the present invention may be used to reduce tension, irritability and stress.

Preferably, the composition contained in the apparatus according to the present invention comprises at least 30 volume %, more preferably at least 50 volume %, yet more preferably at least 60 volume % oxygen. For example, the composition may comprise at least 70 volume % or at least 80 volume % oxygen. In one embodiment, the composition includes greater than 90 volume % oxygen.

The oxygen in the composition may be derived from a pressurised source of substantially pure oxygen gas. For example, the oxygen may be derived from a source of at least 95 volume %, preferably at least 97 volume % oxygen. The oxygen may be stored in a pressurized container either in a conventional canister or with a bag-on-valve canister. Alternatively or additionally, the oxygen may be generated, for example, by a chemical reaction. For instance, oxygen may also be generated chemically from a source of oxygen, such as an inorganic chlorate or perchlorate. Preferably, sodium chlorate is employed. The chemical generation of oxygen may be triggered by an ignition source, such as a spark or electric current. The oxygen source may also be ignited mechanically, for example, on impact using a hammer.

In one embodiment, pellets of sodium chlorate are placed in close proximity to nicotine particles, optionally with a thin barrier positioned therebetween. Once ignited, the sodium chlorate reacts in an exothermic reaction to produce oxygen. The heat generated by the reaction may be used to vaporise the nicotine and, optionally, other additives in the composition. The nicotine vapour and oxygen may be delivered to the user using any suitable apparatus. For example, the apparatus may be provided with a protective heat barrier and a semi-permeable filter. Thus, when the composition is inhaled through the apparatus, for example, through a mouthpiece, the composition is cooled and filtered prior to delivery.

Alternatively, electrochemical oxygen generation is possible. For example, a catalyst, such as cobalt chelate, may be used to store oxygen, and release it when activated by an electric current. This electric current may activate a microprocessor linked to an atomiser which would aerosolise a mixture of nicotine and solutions located within the device. Both oxygen and aerosolised vapour would be drawn though the device by the user's breath on the mouthpiece.

The composition may include less than 75 volume % nitrogen. Preferably, the composition comprises less than 60 volume %, more preferably less than 50 volume % nitrogen. In one embodiment, the composition comprises less than 30 volume %, nitrogen.

The composition may include less than 5 volume % carbon dioxide and/or carbon monoxide. Preferably, the composition includes less than 2 volume %, more preferably less than 0.5 volume %, even more preferably less than 0.04 volume % carbon dioxide and/or carbon monoxide. In a preferred embodiment, carbon monoxide and/or carbon dioxide are substantially absent from the composition.

The composition includes nicotine or a salt thereof in a concentration of 0.0005 to 5 w/w%, preferably 0.001 to 2 w/w%, more preferably 0.001 to 1 w/w%. In preferred embodiments, the composition includes 0.01 w/w%, 0.1 w/w% or 1 w/w% nicotine/nicotine salt.

Optimally, for every 6.6g of pressurised oxygen, 0.01 to 0.5g of nicotine, preferably 0.06 to 0.2g of nicotine (or salt) is employed. The precise amount of nicotine or salt employed may depend on the nature of the nicotine source employed. In the case of a nicotine salt having an acid molar ratio of 1:1, 0.06g of nicotine may be employed for every 6.6 g of pressurised oxygen. This amount of nicotine may be increased if the acid molar ratio of the salt is higher. For example, where the acid molar ratio of the nicotine salt is 2:1 (e.g. in nicotine salts of lauric, citric, malic acid), 0.12g of nicotine may be used for every 6.6g of pressurised oxygen. Where the acid molar ratio of the nicotine salt is 3:1 (e.g. in nicotine salts of proprionic acid), 0.12g of nicotine may be used for every 6.6g of pressurised oxygen.

Any suitable source of nicotine may be employed. For example, the nicotine may be nicotine free base, and/or a nicotine salt. Where a nicotine free base is employed, it may be employed in liquid form. Where a nicotine salt is employed, it may be employed in the form of a solution. Suitable solvents for such solutions include alcohols, glycols and glycol ethers. Specific examples include propylene glycol, polypropylene glycol, polyethylene glycol (PEG), glycerol, ethanol and propanol. Preferably the solvent is polyethylene glycol, and/or propylene glycol. More preferably the solvent is propylene glycol. In one preferred embodiment the solvent comprises ethanol and propylene glycol. The solvent may be present in amounts of 0.1 to 20 w/w%, preferably 1 to 10 w/w%. Preferably nicotine is present in the composition in liquid form.

Suitable nicotine salts include salts formed of the following acids: acetic, proprionic, 1,2-butyric, methylbutyric, valeric, lauric, palmitic, tartaric, citric, malic, oxalic, benzoic, alginic, hydrochloric, chloroplatinic, silicotungstic, pyruvic, glutamic and aspartic. Other nicotine salts, such as nicotine bitartrate dehydrate, may also be employed. Mixtures of two or more nicotine salts may be employed.

The composition may also include an antioxidant. One or more antioxidants may be employed. The precise amount of antioxidant employed may vary depending on the nature of the antioxidant employed. The antioxidant may be present in an amount of 0.0001 to 5 w/w%, preferably 0.001 to 2 w/w%, more preferably 0.001 to 1 w/w% of the composition. In a preferred embodiment, the ratio of nicotine (or salt or derivative thereof) to antioxidant is at least 1.5:1. The ratio of nicotine (or salt or derivative thereof) to antioxidant may be 2:1 or more,; 3:1 or more,; 4:1 or more; 5:1 or more; or even 10:1 or more.

When anti-oxidants, such as carotenoids, are included in the composition, they can undergo a partial degradation in a similar process to carotenoid degradation in tobacco smoke, and thus a similar taste. This makes the composition appealing to the user.

Suitable antioxidants include phenolic compounds, flavonols, flavanones, flavones, flavon-3-ols, anthocyanins(flavonals), isoflavones , coumestans, carotenes, xanthophylls, phytosterols and tocopherols.

Suitable phenolic compounds include monophenols, apiole, carnosol, carvacrol, dillapiole, rosemarinol and flavonoids (polyphenols).

Suitable flavonols include quercetin, gingerol, kaempferol, myricetin, resveratrol and isorhamnetin.

Suitable flavanones include hesperidin, naringenin, silybin and eriodictyol.

Suitable flavones include apigenin, tangeritin and luteolin.

Suitable flavan-3-ols include catechins, catechin, gallocatechin, epicatechin, epigallocatechin, epicatechin 3-gallate, epigallocatechin 3-gallate, theaflavin, theaflavin 3-gallate, theaflavin 3'-gallate, theaflavin 3,3' digallate and thearubigins.

Suitable anthocyanins (flavonals) include pelargonidin, peonidin, cyanidin, delphinidin, malvidin and petunidin.

Suitable isoflavones (phytoestrogens) include daidzein (formononetin).

Suitable coumestans (phytoestrogens) include coumestrol, ellagic acid, gallic acid, salicylic acid, tannic acid, vanillin, capsaicin, curcumin (oxidizes to vanillin), hydroxycinnamic acids, caffeic acid, chlorogenic acid, cinnamic acid, ferulic acid, coumarin, lignans (phytoestrogens), silymarin and matairesinol, secoisolariciresinol, terpenes (isoprenoids) and carotenoids (tetraterpenoids).

Suitable carotenes (orange pigments) include alpha carotene, beta carotene, gamma carotene, delta carotene, lycopene, neurosporene, phytofluene and phytoene.

Suitable xanthophylls (yellow pigments) include canthaxanthin, cryptoxanthin, zeaxanthin, astaxanthin, lutein, monoterpenes, limonene, perillyl alcohol and saponins.

Suitable phytosterols include campesterol, beta sitosterol, gamma sitosterol and stigmasterol.

Omega-3,6,9 fatty acids may also be used as the antioxidant component.

The composition may also include an essential oil. The essential oil may be present in an amount of 0.0001 to 5 w/w%, preferably 0.001 to 2 w/w%, more preferably 0.001 to 1 w/w% of the composition.

Suitable essential oils include geraniol, nerol, phenethyl alcohol, eugenol methyl ether, farrnesol, linalool, citronellol, rose, cuminaldehyde, zingiberene, Farnesol, verbenone, nootkatone, myrcene, menthol citral, citronellol, citronellal, geraniol, nerol, linalool, pinene, thymol and menthone.

The essential oil may be obtained by any suitable means, including distillation, solvent extraction and cold expression.

The composition may include an aroma component. The precise amount of aroma components employed may depend on the nature of the aroma components. The aroma(s) may be present in an amount of 0.0001 to 5 w/w%, preferably 0.001 to 2 w/w%, more preferably 0.001 to 1 w/w% of the composition. In a preferred embodiment, the ratio of nicotine (or salt or derivative thereof) to aroma component is at least 1.5:1. The ratio of nicotine (or salt or derivative thereof) to aroma component may be 2:1 or more,; 3:1 or more,; 4:1 or more; 5:1 or more; or even 10:1 or more.

Suitable aroma components include aromas derived from flowers, leaves or fruit. Preferably, the aroma components are aroma components that are typically added to tobacco products (e.g. accredited aroma components). Specific examples include apple juice concentrate, apricot extract, asafetida, basil oil, balsam peru and oil, angelica root extract, bay leaf sweet oil, bergamot oil, black currant buds absolute, beet juice concentrate, buchu leaf oil, cananga oil, caraway oil, carob bean and extract, beta-carotene, cardamon oleoresin, carob bean and extract, carrot oil, cassia bark oil, cascarilla oil, chicory extract, chocolate, cinnnamon leaf oil, citronella oil, coriander extract and oils, coffee, cognac white and green oil, cubeb oil, costus root oil, dandelion root solid extract, davana oil, fennel sweet oil, fenugreek extract, fig juice concentrate, galbanum oil, genetian root extract, geranium rose oil, ginger oil and oleoresin, glycyrrhizin ammoniated, grape juice concentrate, honey, hops oil, hyssop oil, jasmine absolute concetrate and oil, lavender oil, lemon oil and extract, lemongrass oil, liquorice root, fluid extract and powder, lime oil, lovage oil, mace powder, mandarin oil, maple syrup and concentrate mate leaf, menthol, malt extract, maltol, molasses extract and tincture, oak moss absolute, mullein flowers, myrrh oil, nutmeg powder and oil, oak moss absolute, orange blossoms water, orange oil, origamum oil, orris concentrate oil and root extract, palmarosa oil, parsely seed oil, pepper oil, peppermint oil, pimenta leaf oil, pineapple juice concentrate, pine needle oil, plum juice, prune juice, raisin juice (concentrates), rose absolute and oil, rye extract, rosemary oil, sandalwood oil, spearmint oil, sugars, thyme oil, tocopherols, vanillin, wheat extract, wild cherry bark extract and violet leaf absolute.

The composition may also comprises a flavour component. The flavour component(s) may be present in an amount of 0.0001 to 5 w/w%, preferably 0.001 to 2 w/w%, more preferably 0.001 to 1 w/w% of the composition of the composition.

Suitable flavour components include the flavour components typically added to tobacco products. Examples include carotenoid products, alkenols, aldehydes, esters and delta-lactone flavour constituents.

Suitable carotenoid products include beta ionone, alpha ionone, beta-damascone, beta-damascenone, oxo-edulan I, oxo-edulan II, theaspirone, 4-oxo-beta-ionone, 3-oxo-alpha-ionone, dihydroactinodiolide, 4-oxoisophorone, safranal, beta-cyclocitral.

Suitable alkenols include C₄ to C₁₀ alkenols, preferably C₅ to C₈ alkenols. Specific examples include: cis-2-Penten-1-ol, cis-2-Hexen-1-ol, trans-2-Hexen-1-ol, trans-2-Hexen-1-ol, cis-3-Hexen-1-ol, trans-3-Hexen-1-ol, trans-2-Hepten-1-ol, cis-3-Hepten-1-ol, trans-3-Hepten-1-ol, cis-4-Hepten-1-ol, trans-2-Octen-1-ol, cis-3-Octen-1-ol, cis-5-Octen-1-ol, 1-Octen-3-ol and 3-Octen-2-ol.

Suitable aldehydes include benzaldehyde, glucose and cinnamaldehyde.

Suitable esters include allyl hexanoate, benzyl acetate, bornyl acetate, butyl butyrate, ethyl butyrate, ethyl hexanoate, ethyl cinnamate, ethyl formate, ethyl heptanoate, ethyl isovalerate, ethyl lactate, ethyl nonanoate, ethyl valerate, geranyl acetate, geranyl butyrate, isobutyl acetate, isobutyl formate, isoamyl acetate, isopropyl acetate, linalyl acetate, linalyl butyrate, linalyl formate, methyl acetate, methyl anthranilate, methyl benzoate, methyl benzyl acetate, methyl butyrate, methyl cinnamate, methyl pentanoate, methyl phenyl acetate, methyl salicylate (oil of wintergreen), nonyl caprylate, octyl acetate, octyl butyrate, amyl acetate (pentyl acetate), pentyl hexanoate, pentyl pentanoate, propyl ethanoate, propyl isobutyrate, terpenyl butyrate, ethyl formate, ethyl acetate, ethyl propionate, ethyl butyrate, ethyl valerate, ethyl hexanoate, ethyl heptanoate, ethyl octanoate, ethyl nonanoate, ethyl decanoate, ethyl dodecanoate, ethyl myristate, ethyl palmitate.

Suitable delta-lactone flavour constituents include delta-Hexalactone, delta-Octalactone, delta-Nonalactone, delta-Decalactone, delta-Undecalactone, delta-Dodecalactone, Massoia lactone, Jasmine lactone and 6-Pentyl-alpha-pyrone.

The composition preferably includes a solvent. Suitable solvents include alcohols, glycols and glycol ethers. Specific examples include propylene glycol, polypropylene glycol, polyethylene glycol (PEG) glycerol, ethanol and propanol. Preferably the solvent is Polyethylene glycol (e.g. PEG 400) and/ or propylene glycol. More preferably the solvent is propylene glycol.

The present inventors have found that proplyene glycol is the preferred solvent because it delivers better atomisation characteristics to the aerosol plume compared to other solvents. As a result of the nature of propylene glycol, small diameter aerosol droplets, typically 5 micrometers, can be achieved aiding pulmonary delivery and deposition in the lungs. This means that the absorption profile is faster and more efficient than if larger diameters aerosol droplets are formed. Similarly, because the aerosol is finer using propylene glycol as the solvent, users can experience a mist or vapour that is similar in appearance to smoke.

In one embodiment the solvent may be ethanol and propylene glycol. This further allows for a fine aerosol droplet size to be formed aiding better deposition and distribution.

The solvent may be present in amounts of 0.1 to 20 w/w%, preferably 1 to 10 w/w%. Mixtures of two or more solvents may also be used.

The composition may include cognitive enhancing additives, such as star anise, caffeine, chamomile flower oil, cocoa extract, sage clary oil, eucalyptol, kola nut extract, valerian root extract and powder, green tea, oolong tea, ginkgolides, ginsenosides, taurine, artemisinin, Echinacea, grapefruit mercaptan, lemon oil, cinnamon, tertrahydrocannabinol, cannabidiol and cannabinol. Such cognitive enhancing additives are believed to boost human cognitive abilities i.e. the functions and capacities of the brain. In addition, some of these additives have separate functions, such as antioxidant, aroma, flavour and/or anti-carcinogenic functions.

Such cognitive additives may be present in an amount of 0.0001 to 5 w/w%, preferably 0.001 to 2 w/w%, more preferably 0.001 to 1 w/w% of the composition.

Other optional components include bronchiodilators, such as ophiopogon, glycyrrhizin and theophylline. Where employed, the bronchiodilator may be present in an amount of 0.0001 to 5 w/w%, preferably 0.001 to 2 w/w%, more preferably 0.001 to 1 w/w% of the composition. The ratio of nicotine (or a salt or derivative thereof) to bronchiodilator may be 20:1 or less, for example, 10:1. Where glycyrrhizin is employed, the ratio of nicotine (or a salt or derivative thereof) to glycyrrhizin may be 5:1.

The composition may also include sweeteners, such as natural sugars. Examples include dextrose, fructose, glucose. Sugar alcohols, such as sorbitol, xylitol and glycerol may also be used. Artificial sweeteners may also be employed. Examples include saccharin and its sodium and calcium salts and phenylalanine. Where employed, the sweeteners may be present in an amount of 0.1 to 5 volume % of the composition, for example, 2 to 3 volume % of the composition.

The composition may include stabilizing and/or health enhancing additives, such as vitamins, including vitamin A and C and tocopherols, as well as vitamin D and B12. Where employed, such additives may be present in an amount of 0.1 to 5 volume % of the composition.

For the avoidance of doubt, it should be noted that, while certain additives have been classified in specific categories, certain additives may have more than one function. For example, as well as acting as a cognitive enhancing additive, additives such as green tea may also have anti-carcinogenic properties.

The volume ratio of oxygen to nicotine (or salt or derivative thereof) in the composition of the second aspect of the present invention is at least 1:1, preferably at least 2:1, for example, 1:1 to 3:1.

The pH of the composition is preferably from 5 to 8, more preferably 6 to 7.5, for example approximately 7. Highly acidic and highly alkaline pH's should be avoided to diminish irritation in the mucosal cavity.

The composition may be at least partially hygroscopic. This provides the composition with the ability to attract water molecules from the surrounding environment through either absorption or adsorption. A degree of hygroscopy is desirable as this allows the composition to be deposited further down the airways and reduces the risk of irritation of the glottis.

In a preferred embodiment, the composition comprises:
oxygen,
liquid nicotine or a nicotine salt thereof, and, optionally, at least one of an antioxidant,
an aroma,
a flavour component,
a propellant,
a cognitive enhancing additive, and a solvent.

More preferably, the composition comprises
oxygen,
liquid nicotine or a nicotine derivative or salt thereof,
an antioxidant,
a solvent, and
an aroma.

The use of oxygen can also be of benefit when delivering additives, such as anti-oxidants and anti-carcinogenic compounds, because of the increased transport efficacy of this molecules in oxygen enriched blood.

According to a reference example , there is provided a pressurised container comprising the composition contained in the apparatus of the present invention.

In one embodiment of the reference example, the composition in the pressurised container is a composition according to that contained in the apparatus of the present invention. Alternatively, the composition in the pressurised container may be a composition (e.g. an inhalable composition) comprising oxygen in an amount of less than 22 volume %, and nicotine or a derivative or salt thereof, and a solvent. In one embodiment, the composition contains oxygen in an amount of less than 20 volume%, for example, less than 15 volume%. In another embodiment, the composition contained in the pressurised container comprises oxygen in an amount of at least 1 volume%, at least 2 volume%, at least 5 volume%, or at least 10 volume%. In another embodiment, the pressurised container contains oxygen in an amount of at least 1 volume%, at least 2 volume%, at least 5 volume%, at least 10 volume%, at least 30 volume % or at least 50 volume%. In another embodiment, the composition may contain pressurised air and nicotine or a salt or derivative thereof. For the avoidance of doubt, the optional additives that may be present in the composition of the second aspect of the present invention may also be present in, for example, the corresponding amounts in the composition comprising oxygen in an amount of less than 22 volume %, and nicotine or a derivative or salt thereof.

The pressurised container of the reference example or contained in the apparatus of the present invention may be used to release a gaseous flow of oxygen and liquid nicotine to a user. For example, the pressurised container may be provided with means for delivering the contents of the container to the nasal or oral cavity of a user. Such means may take the form of a button, trigger or breath-activated mechanism. The pressurised container may be used to deliver an unmetered dose of nicotine to the user. This may be advantageous over prior art methods of NRT, such as conventional inhalers, nasal sprays, lozenges and patches currently on the market, because it can allow autonomy in nicotine replacement regulation, where there the user can regulate the amount of compositional nicotine he or she wishes to inhale. Alternatively, or additionally, the pressurised container may be used to deliver a metered dose of nicotine to the user. This may be advantageous so that the user can monitor nicotine intake.

The pressurised container contained in the apparatus of the present invention may be used to release a gaseous flow of oxygen and liquid nicotine to a user without the need for a separate source of energy. For example, the composition may be released without requiring the heating of substrates, combustion of material or a battery powered electric current. Although the container may include an additional propellant (see below), this is also not essential for the release of the composition.

The pressurised container of the present invention may be used in nicotine replacement therapy (NRT). For example, it may be used to deliver nicotine to NRT patients who are advanced smokers and are displaying early or advanced stages of emphysema or chronic obstructive pulmonary disease (COPD). By delivering the oxygen under pressure with nicotine, a patient is required to exert less effort through inhalation over conventional nicotine inhaler, reducing the risk of the patient becoming out of breath. Furthermore, because the flow of oxygen can be substantially constant, users will have no associated problems in co-ordinating the dose.

Another advantage of the pressurised container contained in the apparatus of the present invention is it is physiologically more beneficial that conventional NRT products. Nicotine is already known as a neuroprotector, which has stress-relieving properties. When oxygen and nicotine are used together and administered by pressurized canister, both combine to form a physiologically beneficial inhalation which is a more nuanced and potent stress-reliever. Moreover, the addition of nicotine can act as a neuroprotector against increased oxygen and glucose levels in the brain. Oxygen also targets the same receptors for nicotine take up and it is especially useful as an adjuvant for nicotine replacement therapy.

In the pressurised container contained in the apparatus of the present invention, oxygen is stored with nicotine under pressure. As a result, the user of a pressurised container with oxygen and nicotine will experience the taste of nicotine that has undergone a partial oxidation, rendering it more akin to a tobacco cigarette in taste. Similarly, with a pressurised container, the constant and consistent flow of oxygen and nicotine can be felt in the oral and buccal cavities, and especially on the glottis and upper throat. This has a pleasant replication of the smoke that is felt in this areas upon the inhalation of a tobacco cigarette.

A further advantage of storing nicotine under pressure with oxygen is that there is no risk of liquid nicotine or nicotine particulates evaporating into the ambient environment. This is a problem with capsule or cartridge nicotine used in conventional NRT inhalers, which lose their strength and intensity when the nicotine is exposed to the outside environment. In the pressurized container, there is no contact with ambient oxygen or air, and therefore the mixture delivers a more accurate and consistent dosage.

The pressurised container contained in the apparatus of the present invention may take the form of a pressurised canister, for example, a pressurised aluminium canister. The canister may be fully recyclable and/or reusable. The canister may be refilled as required by a vending machine or a larger container containing the desired composition under a high pressure gradient. In one embodiment, the canister is a 304L aluminium canister. In another it can be a standard aluminium canister which can be sourced from Aerocan, Cebal, 3M or other. In a further embodiment the canister can be made from PET.

The pressurised container may be pressurised to a pressure of from 3 x 10⁵Pa to 1.5 x 10⁷Pa (3 to 150 bar), preferably from 5 x 10⁵Pa to 2 x 10⁶Pa (5 to 20 bar), more preferably from 5.5 x 10⁵Pa to 1 x 10⁶Pa (5.5 to 10 bar), most preferably at approximately 6 x 10⁵Pa (6 bar).

The pressurised container may be capable of dispensing the composition as a mixture of aerosolised solution. Preferably, the mixture has a particle size distribution that is similar to tobacco smoke. The mixture may have the appearance of a vapour or smoke.

The average droplet size in the mixture is preferably from 1 to 40 micrometers, preferably from 2 to 10 micrometers, most preferably approximately 5 micrometers.

The composition may additionally include a propellant, such as a hydrofluorocarbon, preferably a hydrofluoroalkane. Suitable propellants include 1,1,2,2-tetrafluoroethane (HFC-134a) and 1,1,1,2,3,3-heptafluoropropane (HFC-227). The propellant is preferably liquefied. The pressurized container may contain a propellant with oxygen either in the canister or extraneously using a bag-on-valve system.

The composition may comprise at least 1 volume % propellant. Preferably, the composition comprises at least 10 volume % propellant, more preferably at least 30 volume %. In one embodiment the composition comprises more than 50 volume % propellant.

In the pressurised container of the present invention the solvent contained therein is preferably an alcohol, a glycol and/or a glycol ether. Specific examples include propylene glycol, polypropylene glycol, polyethylene glycol (PEG) glycerol, ethanol and propanol. Preferably the solvent is Polyethylene glycol (e.g. PEG 400) and/ or propylene glycol. More preferably the solvent is propylene glycol.

The present inventors have found that proplyene glycol is the preferred solvent because it delivers better atomisation characteristics to the aerosol plume compared to other solvents. As a result of the nature of propylene glycol, small diameter aerosol droplets, typically 5 micrometers, can be achieved aiding pulmonary delivery and deposition in the lungs. This means that the absorption profile is faster and more efficient than is larger diameters aerosol droplets are formed. Similarly, because the aerosol is finer with the user of propylene glycol, users can experience a mist or vapour that is similar in appearance to smoke.

In one embodiment the solvent may be ethanol and propylene glycol. This further allows for a fine aerosol droplet size to be formed aiding better deposition and distribution.

The solvent may be present in amounts of 0.1 to 20 w/w%, preferably 1 to 10 w/w%. Mixtures of two or more solvents may also be used.

In one embodiment of a reference example the pressurized container comprises:
at least 1 volume % oxygen;
a nicotine, a nicotine derivative or salt thereof;
a solvent and
at least 30 volume % of a propellant.

In this embodiment preferably the nicotine is in the liquid form. Preferably the solvent is propylene glycol and/or ethanol. Preferably the propellant is a hydrofluoralkane.

In another embodiment of a reference example the pressurized container comprises:
at least 10 volume % oxygen;
a nicotine, a nicotine derivative or salt thereof;
a solvent and
at least 20 volume % of a propellant.

In this embodiment preferably the nicotine is in the liquid form. Preferably the solvent is propylene glycol and/or ethanol. Preferably the propellant is a hydrofluoroalkane.

In further embodiment of the present invention the pressurized container comprises:
at least 22 volume % oxygen;
a nicotine, or salt thereof;
a solvent and
at least 10 volume % of a propellant.

In this embodiment preferably the nicotine is in the liquid form. Preferably the solvent is propylene glycol and/or ethanol. Preferably the propellant is a hydrofluoroalkane.

The container may be pressurised to a pressure of from 3 x 1.0⁵Pa to 1.5 x 10⁷Pa (3 to 150 bar), preferably from 5 x 10⁵Pa to 2 x 10⁶ Pa (5 to 20 bar), more preferably from 5.5 x 10⁵Pa to 1 x 10⁶Pa (5.5 to 10 bar), most preferably at approximately 6 x 10⁵Pa (6 bar). The pressurised container may be provided with a valve that may be actuated to deliver the composition into the nasal or oral cavity of a user, for example, in the form of mixture of aerosolised particles. This mixture may have the appearance of a smoke or vapour. Because the composition is pressurised, the aerosolised mixture can be produced without the need for a separate power source. As the pressure in the container decreases, the rate of release of the composition from the container may also decrease, replicating the fall in nicotine yields per puff on a tobacco cigarette.

In an alternative embodiment, the composition contained in the apparatus of the present invention is generated *in situ* within the apparatus. For example, the apparatus may include a first reservoir containing oxygen, and a second reservoir containing nicotine or a derivative or salt thereof, and means for mixing said oxygen with said nicotine or derivative or salt derivative thereof to produce the composition contained in the apparatus of the present invention.

The first reservoir may take the form of a pressurised container of oxygen. The container may contain a gas comprising at least 30 volume % oxygen, preferably at least 50 volume %, more preferably at least 70 volume %, and most preferably at least 90 volume % oxygen. In a preferred embodiment the container contains substantially pure oxygen gas.

The second reservoir preferably contains the nicotine or derivative, or salt thereof in liquid or solution form, optionally with the optional additives and components, and may contain solvent and propellant.

The first reservoir may be provided with a valve, which is operable to allow oxygen from the first reservoir to pass through the contents of the second reservoir to produce the composition of the second aspect of the present invention *in situ*. Alternatively, the first reservoir and the second reservoir are provided with their respective valves, which are operable to allow the contents of the first and second reservoir to flow into a mixing chamber in which the composition contained in the apparatus of the present invention is produced *in situ*. The composition produced is delivered into the nasal or oral cavity of a user, for example, in the form of mixture of aerosolised particles. This mixture may have the appearance of a smoke or vapour. Because the pressurised oxygen is employed to produce the composition, an aerosolised mixture can be produced without the need for a separate power source.

In another alternative embodiment, the apparatus comprises a means for generating oxygen, a reservoir containing nicotine or a nicotine derivative or salt, and means for mixing the generated oxygen with said nicotine or derivative or salt thereof to produce the composition of the present invention. As explained above, the means for generating oxygen may take the form of an electrochemical cell. Alternatively, oxygen may be generated chemically from a source of oxygen, such as an inorganic chlorate or perchlorate. The generated oxygen is used to produce the composition contained in the apparatus of the present invention *in situ*. To ensure that the composition is delivered in the form of mixture of aerosolised particles, the apparatus may additionally be provided with a microprocessor, such as a battery-powered microprocessor, to generate the desired smoke or vapour effect.

The apparatus may be provided with a filter or atomiser to aid the dispersion of the composition of the present invention.

The apparatus may also be provided with a heating element to heat the composition prior to delivery.

The apparatus may also be provided with, for example, a heat barrier for reducing the temperature of the composition before it reaches the nasal or oral cavity of the user, if necessary.

The apparatus preferably takes the form of a simulated cigarette device comprising an elongate housing having an inhaling outlet (e.g. mouthpiece) at one end and a reservoir extending along a substantial portion of the housing. The housing preferably has an outlet valve adjacent to the inhaling outlet, which is operable to allow the contents of the reservoir to pass from the reservoir and out of the inhaling outlet. The outlet valve may be breath-activated or may be operable by mechanical means.

In a preferred embodiment, the reservoir contains the composition of the second aspect of the present invention. For example, the reservoir may be a pressurised container containing the composition of the second aspect of the present invention.

Alternatively, the reservoir may contain oxygen. For example, the reservoir may take the form of a pressurised container containing at least 30 volume % oxygen, preferably at least 50 volume %, more preferably at least 70 volume %, and even more preferably at least 90 volume % oxygen. The pressurised container may be pressurised to a pressure of from 3 x 10⁵Pa to 1.5 x 10⁷Pa (3 to 150 bar), preferably from 5 x 10⁵Pa to 2 x 10⁶Pa (5 to 20 bar), more preferably from 5.5 x 10⁵Pa to 1 x 10⁶Pa (5.5 to 10 bar), most preferably at approximately 6 x 10⁵Pa (6 bar). In this embodiment, the cigarette device may further include a second reservoir containing nicotine (or a salt or derivative thereof), optionally in combination with the optional additives and components mentioned above. The reservoir containing oxygen may be provided with a valve, which is operable to allow oxygen to pass through the contents of the second reservoir to produce the composition of the second aspect of the present invention in *situ*. Alternatively, the oxygen-containing reservoir and the second reservoir are provided with their respective valves, which are operable to allow the contents of the reservoirs to flow into a mixing chamber in which the composition of the second aspect of the present invention is produced *in situ*. In the case of the simulated cigarette device, the elongate housing may be provided with a refill inlet and the reservoir may be provided with a refill valve adjacent to the refill inlet. The refill unit may include a composition or oxygen reservoir having an outlet and an associated outlet valve. The refill valve of the cigarette device may be arranged to co-operate with the outlet valve of the refill unit to open a flow path from the reservoir of the refill unit to the reservoir of the cigarette device. The oxygen in the oxygen reservoir of the refill unit may be generated *in situ,* for example, by chemical or electrochemical means.

By providing a cigarette device which is refillable from a refill unit which is in itself refillable, the invention provides a user friendly system. The cigarette device can be made to approximately the size of a known smokable product such as cigarette or cigar, while the refill unit can also be readily portable. For example, it can be of comparable size to a cigarette pack. The user is therefore able to carry the cigarette device and refill unit with the same convenience as carrying a packet of cigarettes.

A combination of the cigarette device and refill unit can have sufficient capacity to last for a number of days of normal use before a refill unit needs to be refilled. The reservoir from which the refill unit itself is refilled could, for example, be a vending machine, or a higher pressure cylinder which the user can keep at home or in their car such that they do not have to carry it with them at all times.

Details of the types of apparatus that may be used to deliver the composition of the present invention are provided, by way of example, in the Applicant's copending patent applications, UK patent application numbers 0712304.5, 0712305.2 and 0712306.0.

### Example 1

A composition was prepared by mixing the components below together:
Nicotine - 0.03g
Oxygen - 0.03g
Aromas - 0.01g
Ethanol - 0.048g
Propylene Glycol - 14.91g
HFA134a - 18.39g
Menthol - 0.01g
Eucalyptus - 0.01g
Citronellol - 0.01g

in a pressurized container of 3.7 x 10⁵ Pa(3.7 bar).

### Example 2

A composition was prepared by mixing the components below together:
Nicotine - 0.03g
Oxygen - 2.75g
Aromas - 0.01g
Ethanol - 0.048g
Propylene Glycol - 9.84g
Vanilla Extract - 0.01g
Cocoa Extract 0.01g
Green Tea Extract - 0.01g
Echninacea Extract - 0.01g
in a pressurized container of 1.22 x 10⁶Pa (12.2bar).

### Example 3

A composition was prepared by mixing the components below together:
Nicotine - 0.03g
Oxygen - 0.03g
Aromas - 0.01g
Ethanol - 0.048g
Propylene Glycol - 14.91g
HFA134a - 18.39g
in a pressurized container of 3.7 x 10⁵ Pa (3.7bar).

### Example 4

A composition was prepared by mixing the components below together:
Nicotine - 0.03g
Oxygen - 2.75g
Aromas - 0.01g
Ethanol - 0.048g
Propylene Glycol - 9.84g
HFA134a - 12.14g in a pressurized container of 12.2 bar.

### Example 5

A composition was prepared by mixing the components below together:
Nicotine - 0.03g
Oxygen - 6.6g
Aromas - 0.01g
Ethanol - 0.048g
Propylene Glycol - 4.97g
HFA134a - 6.06g
in a pressurized container of 2.2 x 10⁶Pa (22 bar).

### Example 6

A composition was prepared by mixing the components below together:
6.6g oxygen
0.18g free nicotine base
2.5g polyethylene glycol
0.45g green tea extract
0.45g oolong tea
0.18g Echinacea extract
0.3g lemon oil
0.18 cocoa extract
0.18g cinnamon oil
0.18g glycyrrhizin

## Claims

1. An apparatus for nicotine delivery containing:
an inhalable composition; and
means for delivering said composition to the nasal or oral cavity of a user,
the inhalable composition comprising:
at least 22 volume % oxygen; and
0.0005 to 5 w/w% nicotine or a nicotine salt.

2. An apparatus as claimed in claim 1, wherein the composition is contained in a pressurised container

3. The apparatus as claimed in claim 2 wherein the container is pressurised to at least 3 x 10⁵ Pa.

4. The apparatus as claimed in any preceding claim, wherein the composition further comprises a solvent, preferably an alcohol, glycol or glycol ether solvent.

5. The apparatus as claimed in any preceding claim, wherein the composition further comprises an antioxidant and/or a cognitive enhancing additive, and/or an essential oil, and/or a flavour and/or aroma component.

6. The apparatus as claimed in any preceding claim wherein the composition comprises a hydrofluoroalkane propellant.

7. An apparatus as claimed in claim 1, which comprises:
a first reservoir containing oxygen,
a second reservoir containing nicotine or a nicotine derivative or salt, and
means for mixing said oxygen with said nicotine or nicotine derivative or salt to produce the composition.

8. An apparatus as claimed in claim 1, which comprises:
means for generating oxygen,
a first reservoir containing nicotine or a nicotine derivative or salt, and
means for mixing the generated oxygen with said nicotine or nicotine derivative or salt to produce the composition.

9. An apparatus as claimed in any preceding claim, which is in the form of a simulated cigarette.

## Patentansprüche

1. Vorrichtung zur Nicotinabgabe, die umfasst:
eine inhalierbare Zusammensetzung; und
eine Einrichtung, um die Zusammensetzung in die Nasenhöhle oder Mundhöhle eines Anwenders zu bringen;
wobei die inhalierbare Zusammensetzung enthält:
mindestens 22 Vol.-% Sauerstoff; und
0,0005 bis 5 Gew.-% Nicotin oder Nicotinsalz.

2. Vorrichtung nach Anspruch 1, wobei die Zusammensetzung in einem unter Druck stehenden Behälter enthalten ist.

3. Vorrichtung nach Anspruch 2, wobei der Behälter auf zumindest 3 x 10⁵ Pa unter Druck gesetzt ist.

4. Vorrichtung nach einem der vorhergehenden Ansprüche, wobei die Zusammensetzung ferner ein Lösemittel, vorzugsweise einen Alkohol, ein Glycol oder einen Glycolether, enthält.

5. Vorrichtung nach einem der vorhergehenden Ansprüche, wobei die Zusammensetzung ferner ein Antioxidationsmittel und/oder einen leitungssteigernden Zusatz (Cognitive Enhancer) und/oder ein etherisches Öl und/oder einen Duftstoff und/oder eine Aromakomponente enthält.

6. Vorrichtung nach einem der vorhergehenden Ansprüche, wobei die Zusammensetzung ferner ein als Treibmittel dienendes Fluorwasserstoffalkan enthält.

7. Vorrichtung nach Anspruch 1, die aufweist:
ein erstes Reservoir, das Sauerstoff enthält,
ein zweites Reservoir, das Nicotin oder ein Nicotinderivat oder
Nicotinsalz enthält, und
eine Einrichtung zum Mischen des Sauerstoffs mit dem Nicotin oder Nicotinderivat oder Nicotinsalz zur Bildung der Zusammensetzung.

8. Vorrichtung nach Anspruch 1, die aufweist:
eine Einrichtung zur Bildung von Sauerstoff,
ein erstes Reservoir, das Nicotin oder ein Nicotinderivat oder
Nicotinsalz enthält, und
eine Einrichtung zum Mischen des gebildeten Sauerstoffs mit dem Nicotin oder Nicotinderivat oder Nicotinsalz zur Bildung der Zusammensetzung.

9. Vorrichtung nach einem der vorhergehenden Ansprüche, die in Form einer simulierten Zigarette vorliegt.

## Revendications

1. Dispositif pour délivrer de la nicotine contenant:
une composition inhalable ; et
des moyens pour délivrer ladite composition à la cavité nasale ou orale d'un utilisateur,
la composition inhalable comprenant :
au moins 22 % en volume d'oxygène ; et
0,0005 à 5 % en poids/poids de nicotine ou d'un sel de nicotine.

2. Dispositif selon la revendication 1, dans lequel la composition est contenue dans un récipient pressurisé.

3. Dispositif selon la revendication 2, dans lequel le récipient est pressurisé sous au moins 3 x 10⁵ Pa.

4. Dispositif selon l'une quelconque des revendications précédentes, dans lequel la composition comprend en outre un solvant, de préférence un solvant de type alcool, glycol, ou éther de glycol.

5. Dispositif selon l'une quelconque des revendications précédentes, dans lequel la composition comprend en outre un antioxydant et/ou un additif amplifiant les fonctions cognitives, et/ou une huile essentielle et/ou un parfum et/ou un composant aromatique.

6. Dispositif selon l'une quelconque des revendications précédentes, dans lequel la composition comprend un propulseur de type hydrofluoroalcane.

7. Dispositif selon la revendication 1, qui comprend:
un premier réservoir contenant de l'oxygène,
un deuxième réservoir contenant de la nicotine ou un dérivé ou sel de nicotine, et
des moyens pour mélanger ledit oxygène avec ladite nicotine ou ledit dérivé ou sel de nicotine pour produire la composition.

8. Dispositif selon la revendication 1, qui comprend:
des moyens pour générer de l'oxygène,
un premier réservoir contenant de la nicotine ou un dérivé ou sel de nicotine, et
des moyens pour mélanger l'oxygène généré avec ladite nicotine ou ledit dérivé ou sel de nicotine pour produire la composition.

9. Dispositif selon l'une quelconque des revendications précédentes, qui a la forme d'une cigarette simulée.
